# EUROPÄISCHE PATENTANMELDUNG

(11) **EP 4 344 637 A1**
(43) Veröffentlichungstag der Anmeldung: **03.04.2024**
(21) Anmeldenummer: 23199590.3
(22) Anmeldetag: 26.09.2023
(51) Int. Cl.: A61B 5/20, A61B 5/00

(54) **VORRICHTUNG ZUM MESSEN EINES FLUSSES EINER FLÜSSIGKEIT, INSBESONDERE EINES URINFLUSSES**

(30) Priorität: 26.09.2022 DE 102022124611
(71) Anmelder: Sonotec GmbH, 06112 Halle (Saale) (DE); Wiegand, Stefan, 06114 Halle (DE)
(72) Erfinder: WIEGAND, Stefan, 06114 Halle (DE); MÜNCH, Hans-Joachim, 06130 Halle (Saale) (DE); SCHAICH, Hans-Christian, 04155 Leipzig (DE)
(74) Vertreter: Maikowski & Ninnemann Patentanwälte Partnerschaft mbB

(57) **Zusammenfassung**

Eine Vorrichtung zum Messen eines Flusses einer Flüssigkeit in Abhängigkeit von der Zeit, insbesondere eines Urinflusses, weist auf:
• einen Einlauftrichter;
• einen Schlauch;
• einen Ultraschall Durchflusssensor;
• einen Überlauf.

Ein erstes Ende des Schlauchs ist mit dem Einlauftrichter verbunden ist und ein zweites Ende des Schlauchs mit dem Überlauf;
der Ultraschall Durchflusssensor ist am Schlauch angeordnet; der Schlauch ist U-förmig ausgebildet.

## Beschreibung

Die Erfindung betrifft eine Vorrichtung zum Messen eines Flusses einer Flüssigkeit in Abhängigkeit von der Zeit, insbesondere eines Urinflusses, aufweisend
- einen Einlauftrichter;
- einen Schlauch;
- einen Ultraschall Durchflusssensor;
- einen Überlauf.

Die Vorrichtung ist dadurch gekennzeichnet, dass
ein erstes Ende des Schlauchs mit dem Einlauftrichter verbunden ist und ein zweites Ende des Schlauchs mit dem Überlauf;
der Ultraschall Durchflusssensor am Schlauch angeordnet ist;
der Schlauch U-förmig ausgebildet ist.

Insbesondere in der Urologie wird dies vor allem zur Diagnostik von Harnwegsobstruktionen oder neurologischen Funktionsstörungen des Schließmuskels genutzt. Aus den sich ergebenden Diagrammen und Kennwerten (V(t) / Vmax / t) lassen sich Rückschlüsse auf Ursachen von Beschwerden ziehen bzw. Diagnosen erstellen.

Für die Erfüllung dieser Aufgabe kommen aktuell auf unterschiedlichste Weise gestaltete Wäge Konzepte zum Einsatz. Im Kern wird dabei immer der Urin in ein auf einer empfindlichen Waage stehendes Auffanggefäß umgeleitet. Durch das Auslesen der gemessenen Masse über die Zeit und der Berechnung des Volumens über die Dichte lässt sich ein Flussdiagramm erstellen. Viele Patente beschäftigen sich mit der Optimierung dieses Wägeprinzips zur Eliminierung von Parametern, die die Messung stören. Beispielhaft sind hier die DE 19 61 33 06 C2, GB 24 37 549 A, DE 300 78 55 C2 und die EP 2 564 778 B1 zu nennen.

Die EP 2 564 778 B1 betrifft eine Vorrichtung und ein Verfahren zur Harnflussmessung, die möglichst einfach und wartungsfrei gestaltet sein soll, um auch im nichtmedizinischen Umfeld eingesetzt werden zu können. Dass Messprinzip besteht aus einem Messbecher, der gegenüber einem Messwertgeber derart angeordnet ist, dass eine Verschiebung des Messbechers gegenüber dem Messwertgeber als Maß für das Volumen von in dem Messbecher gesammeltem Urin detektierbar ist. Die Verschiebung des Messbechers wird dabei durch die Schwerkraft, die auf den gesammelten Urin wirkt hervorgerufen.

Die Nutzung einer Waage als Mittel zur Messwerterfassung stellt in der Praxis häufig ein Problem dar. Um die Validität der gelieferten Messwerte des empfindlichen Messmittels sicherzustellen, muss die Waage regelmäßig kalibriert werden. Dies führt zu Kosten und Ausfallzeiten der Messeinrichtung für die Uroflowmetrie. Auch im Alltag von Praxen und Krankenhäusern besteht die Gefahr, dass das Messmittel durch inadäquates handling Schaden nimmt - die Validität der Messwerte ist dann schlimmstenfalls nicht mehr gegeben.

Der Erfindung liegt daher die Aufgabe zu Grunde, die Nachteile des Standes der Technik zu beheben und eine Vorrichtung zum Messen eines Flusses einer Flüssigkeit in Abhängigkeit von der Zeit, insbesondere eines Urinflusses zur Verfügung zu stellen, die äußerst robust, einfach in der Anwendung und dabei verlässlich valide Messergebnisse liefert.

Diese Aufgabe wird von der Vorrichtung gemäß Anspruch 1 gelöst. Weiterhin wird die Verwendung der Vorrichtung zur Messung eines Urinflusses pro Zeit beschrieben.

### Detaillierte Beschreibung

Die vorliegende Erfindung betrifft eine Vorrichtung zum Messen eines Flusses einer Flüssigkeit in Abhängigkeit von der Zeit, insbesondere eines Urinflusses.

Die Flüssigkeit, deren Fluss gemessen wird, weist bevorzugt eine Dichte von 0,95 - 1,05 g/ml, eine Temperatur von 10-40 °C und eine dynamische Viskosität von 0,6-1,5 mPa*s auf. Besonders bevorzugt ist die Flüssigkeit Urin oder ein Urin-Wasser Gemisch mit einer Dichte von 1,00-1,05 g/, einer Temperatur von 10-40 °C und einer dynamischen Viskosität von 0,6-1,5 mPa*s.

Die erfindungsgemäße Vorrichtung ermöglicht insbesondere die Messung des Flusses von Volumenströmen von Flüssigkeiten zwischen 180 ml/min bis 4200 ml/min.

Die Vorrichtung weist erfindungsgemäß einen
- einen Einlauftrichter;
- einen Schlauch;
- einen Ultraschall Durchflusssensor; und
- einen Überlauf;
auf.

Der Einlauftrichter hat die Aufgabe die Flüssigkeit, insbesondere den Urin, vollständig aufzufangen und in den daran angeschlossenen Schlauch abzuleiten. Seine Form hat dabei einen Einfluss darauf, ob sich im Schlauch Strudel bilden, welche die Messung beeinträchtigen können. An sich sind Einlauftrichter aus dem Stand der Technik bekannt, sie weisen einen Teil auf, der konisch Verläuft und in einen Ablauf mündet. Ein für die erfindungsgemäße Vorrichtung geeigneter Einlauftrichter weist einen Durchmesser der Öffnung des konischen Teils zwischen 5 cm und 30 cm auf.

In einer Ausführungsform der vorliegenden Erfindung weist der Einlauftrichter konzentrische auf den Ablauf zulaufenden Strömungsbrecher und/oder einen abgeknickten Ablauf auf. Am Ablauf ist ein erstes Ende eines Schlauches am Einlauftrichter angeschlossen.

Weist der Einlauftrichter konzentrische auf den Ablauf zulaufende Strömungsbrecher auf, können diese in Form von Stegen quaderförmigen oder runden Querschnitts auf den konischen Flächen des Einlauftrichters angeordnet sein. Eine maximale Anzahl von Strömungsbrechern im Einlauftrichter ist nicht zu definieren. In einer Ausführungsform der Erfindung weist der Einlauftrichter 1, 2, 3, 4, 5, 6, 7, 8, 9 oder 10 Strömungsbrecher auf. Auch eine zum Zentrum hin geriffelte Oberfläche kann die Strudelbildung effektiv verhindern.

Weist der Einlauftrichter einen abgeknickten Ablauf auf, weicht die Achse des abgeknickten Auslaufs des Einlauftrichters von der Achse des konischen Teils des Einlauftrichters ab. Dadurch werden eventuell entstehende Strudel zuverlässig aufgebrochen. Eine im Inneren des Strudels entstehende dünne Luftsäule wird verhindert.

In einer weiteren Ausführungsform der vorliegenden Erfindung weist der Einlauftrichter eine Gitterstruktur im Auslauf des Trichters auf. Das heißt die Gitterstruktur ist vor dem angeschlossenen Schlauch im Einlauftrichter angeordnet.

Die beschriebenen Ausführungsformen des Einlauftrichters sind dazu geeignet eine störende Strudelbildung zu verhindern.

Erfindungsgemäß ist wie bereits beschrieben ein erstes Ende des Schlauchs mit dem Einlauftrichter verbunden. Diese Verbindung kann durch alleiniges Überstülpen des Schlauches über den Trichterauslass oder andersherum, sowie durch eine Klemmung des Schlauches auf dem Trichterauslass mittels Schlauchklemme hergestellt werden. Der Durchmesser des Ablaufs ist daher so gestaltet, dass das erste Ende des Schlauchs entsprechend damit verbunden werden kann.

Der Einlauftrichter sollte ein Material aufweisen oder aus einem Material bestehen, welches eine glatte Oberfläche des Einlauftrichters gewährleistet und eine Beständigkeit gegenüber Reinigungschemikalien aufweist. Der Einlauftrichter kann daher Materialien aufweisen oder aus Materialien hergestellt sein enthalten in der Gruppe aufweisend Keramik, Emaille, Edelstahl und Aluminium. In einer Ausführungsform weist der Einlauftrichter einen inerten Kunststoff ausgewählt aus der Gruppe aufweisend Polyethylen (PE), Polypropylen (PP), Polyethylenterephthalat (PET), Polyamide (PA), Acrylnitril-Butadien-Styrol (ABS), Polytetrafluorethylen (PTFE). In einer Ausführungsform der Erfindung weisen lediglich die Oberflächen, die mit der Flüssigkeit in Kontakt kommen die beschriebenen Materialien auf.

Am Einlauftrichter ist ein erstes Ende des Schlauches angeordnet und das zweite Ende des Schlauches ist erfindungsgemäß mit dem Überlauf verbunden. Die Verbindung mit dem Überlauf kann dabei durch alleiniges Überstülpen des Schlauches über den Überlauf oder andersherum, sowie durch eine Klemmung des Schlauches auf dem Überlauf mittels Schlauchklemme hergestellt werden. Der Schlauch verläuft dabei erfindungsgemäß U-förmig. U-förmig beschreibt dabei einen Verlauf des Schlauches, bei dem das erste und das zweite Ende des Schlauches in vertikaler Richtung nach oben gebogen sind. Die Biegung muss dabei nicht zwangsläufig 90° betragen. Jedoch sind die beiden Enden des Schlauchs höher angeordnet als dessen Mittelteil, was einer U-Form entspricht. Das erste und das zweite Ende des Schlauches müssen sich dabei auch nicht auf der gleichen Höhe befinden.

Weiterhin ist erfindungsgemäß der Ultraschall Durchflusssensor am Schlauch angeordnet. Besonders bevorzugt ist der Ultraschall Durchflussensor am Schlauch angeklemmt. Dies ist durch einen sogenannten Clamp-On Durchflusssensor möglich.

In einer bevorzugten Ausführungsform wird die Schlauchstrecke vor dem Eintritt in den Sensor in einer Ausführungsform auf einer Länge von mindestens 10 x ID vor dem Ultraschall Durchflusssensor und auf einer Länge von mindestens 5 x ID hinter dem Ultraschall Durchflusssensor gerade gehalten. Gerade gehalten bedeutet in diesem Zusammenhang, dass die gerade Schlauchstrecke in einem Winkel von 0° bis ±30° zur Horizontalen ausgebildet ist. Diese gerade Ausführungsform gewährleistet, dass Störungen des Flussprofils vermieden werden und damit eine sichere Messung ermöglicht wird. ID bezeichnet den Innendurchmesser des Schlauchs.

Erfindungsgemäß wird zur Durchführung der Flussmessung ein Ultraschall Durchflusssensor genutzt. Aufgrund seiner nicht-invasiven Funktionsweise ist dieser besonders gut geeignet. Es gibt kein anderes Messverfahren, das eine Flussmessung nicht-invasiv mit einer Genauigkeit von bis zu 2% und besser durchführen könnte. Eine initiale Justierung des Sensors oder eine selbstständige Anpassung auf die Schlauchparameter ist vorteilhaft, um die gewünschte Genauigkeit zu erreichen. Eine solche Kalibrierung ist vorteilhafterweise nur einmal notwendig, da die aus dem Stand der Technik bekannte Messtechnik in diesem Bereich ausreichend robust ist.

In einer Ausführungsform der vorliegenden Erfindung zeichnet sich der Ultraschall Durchflusssensor insbesondere dadurch aus, dass er am vollständig mit Flüssigkeit gefüllten Schlauch ohne ein Koppelmittel angeklemmt wird. Bevorzugt werden bei der Messung Ultraschallsignale mit einer Frequenz zwischen 1 MHz und 4 MHz verwendet. Die Messung des Flusses der Flüssigkeit kann damit nicht-invasiv, also ohne Kontakt zur Flüssigkeit im Schlauch mit einer Genauigkeit von ±15 %, bevorzugt von ±5 %, besonders bevorzugt von ±2 % gemessen werden.

In einer weiteren Ausführungsform der vorliegenden Erfindung verfügt der Ultraschall Durchflusssensor über mindestens eine analoge und/oder digitale Schnittstelle. Analoge Schnittstellen können beispielsweise ein Stromausgang, Frequenzausgang, PWM oder ähnliches sein. Digitale Schnittstellen ermöglichen eine serielle Kommunikation über ein standardisiertes oder proprietäres Protokoll. Über diese mindestens eine Schnittstelle können die Messwerte an eine Einheit zum Auswerten weitergegeben werden. Eine Einheit zum Auswerten ist beispielsweise ein PC oder ähnliches. In dieser Einheit können die Messwerte weiterverarbeitet werden.

In einer Ausführungsform weist die vorliegende Erfindung eine Schnittstelle auf, welche eine kabellose Fernübertragung der Messdaten ermöglicht. Die kann über eine WLAN-Schnittstelle und/oder eine Schnittstelle zu einem Mobilfunknetz oder ähnlichem umgesetzt sein. Vorteilhafterweise können die Messdaten so Fernübertragen werden und die erfindungsgemäße Vorrichtung kann auch außerhalb von Arztpraxen, zum Beispiel bei einem Patienten zu Hause oder in einer Pflegeinrichtung selbstständig verwendet werden.

Die Messdaten könne dann jederzeit automatisch und ortsunabhängig zum Arzt gesendet und von diesem analysiert werden.

In einer bevorzugten Ausführungsform der vorliegenden Erfindung weist die erfindungsgemäße Vorrichtung weiterhin eine Batterie oder einen Akku auf. Hierdurch wird die Vorrichtung mit dem notwendigen Strom versorgt. Die erfindungsgemäße Vorrichtung kann dann vorteilhafterweise autark, d.h. unabhängig von einer stationären Stromquelle betrieben werden.

Ein für die erfindungsgemäße Vorrichtung geeigneter Schlauch muss so ausgeführt sein, dass ein Durchfluss von bis zu 4 l/min (bei gesunden Menschen in jungem Erwachsenenalter) ohne nennenswertes Aufstauen hindurchlaufen kann. Ein Aufstauen würde zu einer Verzögerung der Messung des Flusses und damit zu einer Verfälschung des Ergebnisses führen. Allerdings reduziert sich die absolute Messgenauigkeit von Ultraschall Clamp-On Durchflusssensoren mit Erhöhung des Schlauchinnendurchmessers. Grund hierfür ist, dass der Sensor lediglich eine Fließgeschwindigkeit misst, die über eine Faktor Multiplikation zu einem Volumenstrom umgerechnet wird. Demzufolge wären kleine Durchflüsse am besten an dünnen Schläuchen zu messen, da hier die Fließgeschwindigkeit bei kleinen Durchflüssen höher ist als bei dickeren Schläuchen. Bei dickeren Schläuchen reduziert sich also bei gleichbleibendem Volumenstrom der Messeffekt und dadurch auch die Messgenauigkeit. Aus diesen Gründen muss ein Optimum zwischen dickem Schlauch (reelle Darstellung hoher Durchflüsse) und dünnem Schlauch (hohe Genauigkeit bei geringen Durchflüssen) gefunden werden.

Es hat sich gezeigt, dass besonders Schläuche mit einem Innendurchmesser (ID) im Bereich von 3/8" ± 50 %, bevorzugt im Bereich von 3/8" ± 25 %, besonders bevorzugt von 3/8" diesen Ansprüchen genügen und daher in einer Ausführungsform der vorliegenden Erfindung verwendet werden.

In einer Ausführungsform der Erfindung weist der Schlauch weiterhin eine Wandstärke im Bereich von 3/32" ±50 %, bevorzugt im Bereich von 3/32" ± 25 %, besonders bevorzugt von 3/32" auf. Diese Wandstärken haben sich als vorteilhaft erwiesen, um eine möglichst hohe Messgenauigkeit zu erhalten.

In einer Ausführungsform ist der Schlauch ein Polymerschlauch. Bevorzugt ein flexibler Polymerschlauch. Flexibel bedeutet in diesem Zusammenhang, dass der Schlauch durch rein mechanische Krafteinwirkung in die gewünschte U-Form gebogen werden kann. In einer besonders bevorzugten Ausführungsform weist der Schlauch ein Material ausgewählt aus der Gruppe aufweisend Silikon und PVC auf.

In einer Ausführungsform der vorliegenden Erfindung weist die Vorrichtung weiterhin ein Ablassventil auf, welches zwischen dem Ultraschall Durchflusssensor und dem Überlauf am Schlauch angeordnet ist. Es befindet sich bevorzugt an der Tiefsten Stelle des U-Förmig angeordneten Schlauchs. Es ermöglicht bei Bedarf das Entleeren des Systems zum Beispiel zum Ende eines Arbeitsstages, nach einem Messvorgang oder zur Wartung der Vorrichtung. Das Ablassventil kann dabei vor oder nach dem Ultraschall Durchflussensor angeordnet sein. Vorteilhaft ist es, wenn das Ablassventil nicht zu nahe an der Messstelle des Ultraschall Durchflusssensors angeordnet ist. In einer Ausführungsform der erfindungsgemäßen Vorrichtung beträgt der Abstand zwischen Ultraschall Durchflusssensor und Ablassventil mindestens das 10-fache des Schlauchinnendurchmessers. Der Abstand wird dabei zwischen der Kante des Ultraschall Durchflusssensors die dem Ablassventil zugewandt ist der Kante des Ablassventils die dem Ultraschall Durchflussensor zugewandt ist gemessen.

Erfindungsgemäß weist die Vorrichtung einen Überlauf auf. Der Überlauf hat einen entscheidenden Einfluss auf die Genauigkeit des Messsystems. Es ist darauf zu achten, dass eine Sogwirkung durch das auslaufende Medium auf das im Schlauch befindliche Medium unterbunden wird. In einer Ausführungsform der vorliegenden Erfindung weist das zweite Ende des Schlauchs daher eine vertikale, nach oben gerichtete Auslassöffnung im Überlauf auf. Diese Ausgestaltung unterbindet eine Sogwirkung, was bei einem horizontalen oder nach unten gerichteten Schlauchauslauf nicht gewährleistet werden kann.

In einer weiteren Ausführungsform weist die Vorrichtung weiterhin einen Auffangbehälter auf, der nach dem Überlauf angeordnet ist. In einer alternativen Ausführungsform mündet der Überlauf in einen Abfluss. In beiden Ausführungsformen ist gewährleistet, dass die Flüssigkeit, die am Überlauf aus dem Schlauch tritt, nicht am Schlauch herunterläuft. Dies sollte aus hygienischen Gründen vermieden werden. Darüber hinaus wird eine fachgerechte Entsorgung der Flüssigkeit auf diese Weise ermöglicht.

Der Auffangbehälter kann Teil einer den Einlauftrichter, den Schlauch, den Ultraschall Durchflusssensor und den Überlauf ganz oder teilweise umhüllenden Vorrichtung sein oder auch lose mit dem Überlauf verbunden sein. Ist der Auffangbehälter lose mit dem Überlauf verbunden, kann die darin aufgefangene Flüssigkeit vorteilhafterweise weiteren Untersuchungen zugeführt werden. Unter der den Einlauftrichter, den Schlauch, den Ultraschall Durchflusssensor und den Überlauf ganz oder teilweise umhüllenden Vorrichtung ist eine Umhüllung zu verstehen, die die Komponenten umhüllt, wodurch eine kompakte Form der erfindungsgemäßen Vorrichtung entsteht. Unter kompakter Form ist zu verstehen, dass die einzelnen Komponenten so platzsparend wie möglich in der Umhüllung angeordnet sind. Hierdurch kann die Vorrichtung von einem Nutzer leicht bewegt werden. Bei der Verwendung in Pflegeeinrichtungen oder zu Hause ist darüber hinaus der Transport leicht möglich und auch das Lagern der erfindungsgemäßen Vorrichtung, wenn diese nicht genutzt wird, nimmt wenig Platz ein.

In einer Ausführungsform weisen daher Teile der Vorrichtung oder die gesamte Vorrichtung eine Umhüllung auf.

In einer weiteren Ausführungsform weist die Vorrichtung weiterhin eine Stuhlkonstruktion oder ein Urinal auf, wobei die Stuhlkonstruktion oder das Urinal mit dem Einlauftrichter verbunden ist, oder der Einlauftrichter durch die Stuhlkonstruktion oder das Urinal abgebildet wird, oder die genannte Vorrichtung in kompakter Bauform in einer Toilette eingehängt wird. In diesen Ausführungsformen kann der Auffangbehälter beispielsweise durch das Urinal oder die Stuhlkonstruktion gebildet werden. In diesem Fall umhüllt der Auffangbehälter ganz oder teilweise den Einlauftrichter, den Schlauch, den Ultraschall Durchflusssensor und den Überlauf der Vorrichtung.

Eine Stuhlkonstruktion kann beispielsweise ein handelsüblicher Toilettenstuhl sein, wie er im Pflegebereich und in Kliniken Verwendung findet.

Weiterhin wird die Verwendung der Vorrichtung gemäß einem der vorangegangenen Ansprüche zur Messung eines Urinflusses pro Zeit beschrieben. Insbesondere für den Einsatz in urologischen Praxen oder Kliniken ist die erfindungsgemäße Vorrichtung geeignet. Auch eine Heimanwendung ist mit der erfindungsgemäßen Vorrichtung möglich.

Die Messung eines Flüssigkeitsflusses, insbesondere eines Urinflusses mit der erfindungsgemäßen Vorrichtung erweist sich als äußerst robust. Die Nutzung eines Ultraschall Durchflusssensors an Stelle einer Waage behebt die im Stand der Technik genannten Unzulänglichkeiten. Insbesondere die Nutzung eines nicht-invasiven Ultraschall Durchflusssensors verbessert das Handling und die Robustheit der Messung ungemein. Ein Clamp-On Durchflusssensor ist in der Lage, durch die Wandung eines Kunststoff Schlauchs (z.B. Silikon, PVC oder ähnlichen) den Volumenstrom des Flüssigkeitsstroms, insbesondere eines Urinstroms im Schlauch zu messen. Die auf Ultraschall basierende Technologie misst schon kleinste Flüsse zuverlässig und mit hoher Präzision. Ein für die Messmethode angepasster Aufbau stellt zu jeder Zeit valide und genaue Messergebnisse sicher. Eine Re-Kalibrierung des Durchflusssensors ist aufgrund der Verschleißfreiheit und Robustheit der Clamp-On Sensorik nicht nötig. Das Anklemmen eines solchen Sensors an eine Schlauchleitung und auch dessen Nutzung ist für ungeübtes Personal ohne nennenswerte Schulungen möglich.

Im Folgenden wird die Erfindung anhand von 6 Figuren und einem Beispiel näher erläutert.
- Figur 1: stellt einen Aufbau der erfindungsgemäßen Vorrichtung dar;
- Figur 2: stellt einen Aufbau der erfindungsgemäßen Vorrichtung mit abgeknickten Einlauftrichter dar;
- Figur 3: (A) und (B) stellen einen Aufbau der erfindungsgemäßen Vorrichtung mit einer Stuhlkonstruktion dar;
- Figur 4: (A) und (B) stellen den Volumenstrom über die Zeit für zwei verschiedene Volumenströme dar;
- Figur 5: (A) stellt die absolute Abweichung der Messwerte einer Messung mit der erfindungsgemäßen Vorrichtung zu einer Wägemessung dar; (B) stellt die relativen Abweichungen der Messergebnisse dar;
- Figur 6: (A) stellt die absolute Abweichung der Messwerte einer Messung mit der erfindungsgemäßen Vorrichtung zu einer Wägemessung für verschiedene Volumenströme dar; (B) stellt die relative Abweichung der Messergebnisse dar.
- Figur 7: stellt eine Messung eines sich mit der Zeit verändernden Volumenstromes dar.

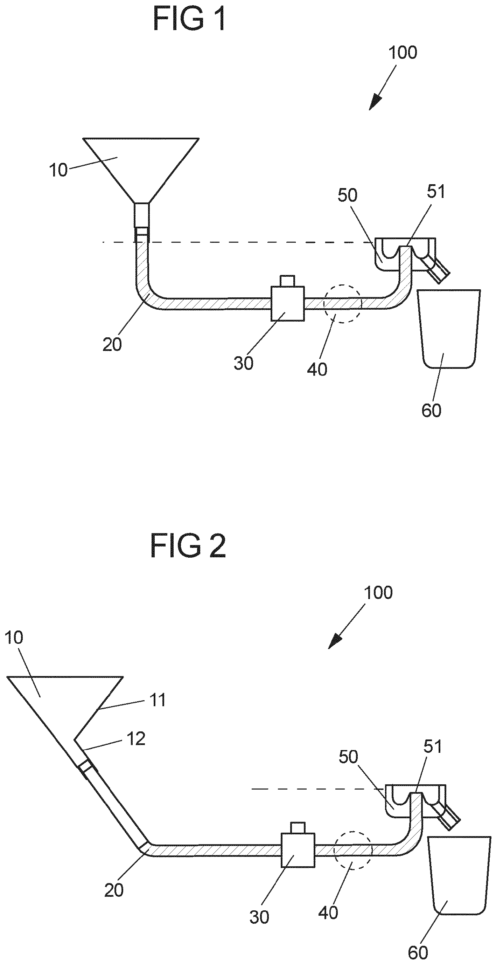

**Figur 1** stellt einen Aufbau der erfindungsgemäßen Vorrichtung 100 dar. Der Einlauftrichter 10 ist mit dem ersten Ende des Schlauches 20 verbunden. Am Schlauch 20 ist der Ultraschall Durchflusssensor 30 angeklemmt. Nach dem Ultraschall Durchflusssensor 30 ist ein Ablassventil 40 angeordnet, mit dem die Vorrichtung 100 bei Bedarf entleert werden kann. Das zweite Ende des Schlauches mündet in den Überlauf 50. Das zweite Ende des Schlauchs weist dabei eine vertikale, nach oben gerichtete Auslassöffnung 51 im Überlauf auf. Die nach oben gerichtete Auslassöffnung wird dabei durch das zweite Ende des Schlauches gebildet, das nach oben gerichtet ist. Zusätzlich weist die Vorrichtung 100 einen Auffangbehälter 60 in Form eines Gefäßes auf.

**Figur 2** stellt eine weitere Ausführungsform der erfindungsgemäßen Vorrichtung 100 dar. Die Vorrichtung 100 weist den gleichen Aufbau wie zu Figur 1 beschrieben auf. Lediglich der Einlauftrichter 10 ist anders als in Figur 1 ausgebildet. Der Einlauftrichter 10 weist einen abgeknickten Auslauf 12 auf, dadurch weicht die Achse des abgeknickten Auslaufs 12 des Einlauftrichters von der Achse des konischen Teils des Einlauftrichters 11 ab. Die Achsen sind in der Figur mit gestrichelten Linien dargestellt.

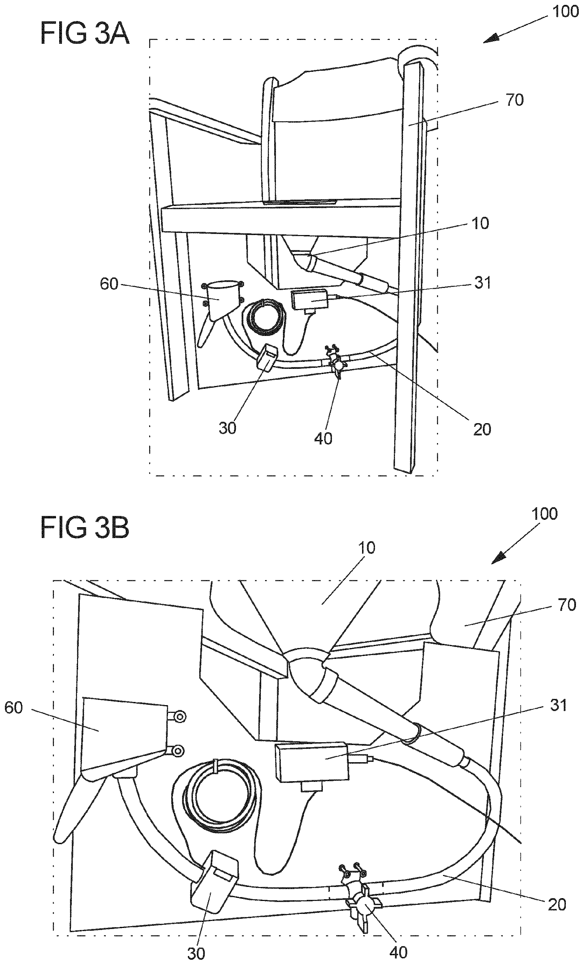

**Figur 3 (A)** stellt einen Aufbau der erfindungsgemäßen Vorrichtung 100 mit einer Stuhlkonstruktion 70 dar. **Figur 3 (B)** zeigt einen vergrößerten Ausschnitt des Aufbaus der erfindungsgemäßen Vorrichtung 100. Die Stuhlkonstruktion 70 ist ein Toilettenstuhl, wie er aus dem Fachhandel für Sanitätsprodukte bekannt ist. Unter dem Toilettenstuhl sind die weiteren Bestandteile der Vorrichtung angebracht. In der Öffnung in der Sitzfläche des Toilettenstuhls ist der Einlauftrichter 10 angeordnet. Der Einlauftrichter 10 ist mit dem ersten Ende des Schlauches 20 verbunden. Am Schlauch 20 sind das Auslassventil 40 und der Ultraschall Durchflusssensor 30 angeordnet. Der Ultraschall Durchflusssensor 30 ist mit einem Datenkonverter 31 verbunden, der wiederum eine Verbindung zu einem PC (nicht abgebildet) ermöglicht. Das zweite Ende des Schlauches 20 mündet in den Überlauf 60.

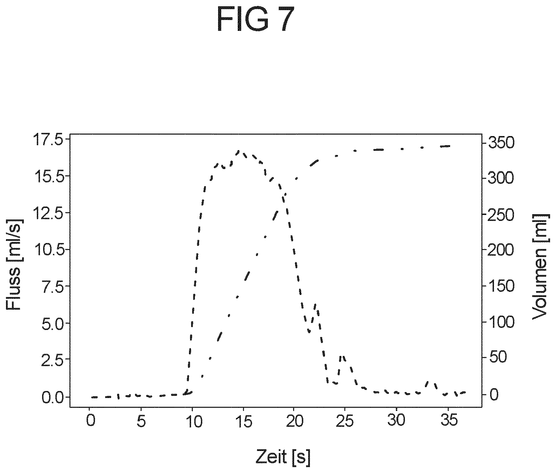

**Figur 7** stellt die Messergebnisse einer Messung eines sich zeitlich verändernden Volumenstromes dar. Die ansteigende und wieder abfallende Kurve zeigt den Fluss in ml/s über der Zeit an. Die ansteigende und dann konstant verlaufende Kurve stellt das gemessene Gesamtvolumen in Abhängigkeit von der Zeit dar.

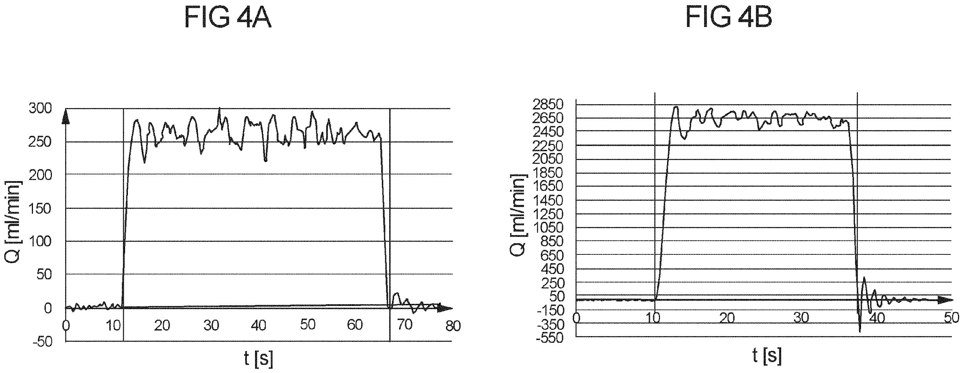

Figuren 4 bis 6 werden im Zusammenhang mit dem Ausführungsbeispiel näher erläutert.

### Ausführungsbeispiel 1

Zur Validierung der erfindungsgemäßen Vorrichtung wurden Vergleichsmessungen durchgeführt. Dazu wurde mit einer Pumpe ein konstanter Volumenstrom (Wasser, 23°C) erzeugt und mit einem Schlauch in den Trichter geleitet. Der Schlauch wurde dabei nicht stillgehalten, sondern die Einlaufposition ständig etwas variiert, um einen Urinierprozess zu simulieren. Es wurden dabei definierte Durchflüsse zwischen 180 ml/min und 2560 ml/min erzeugt. Ein Referenzsensor maß den erzeugten Durchfluss vor Austritt in die erfindungsgemäße Vorrichtung. Nach dem Durchlauf durch die erfindungsgemäße Vorrichtung wurde das Wasser zur Wägung in einem Auffangbehälter aufgefangen.

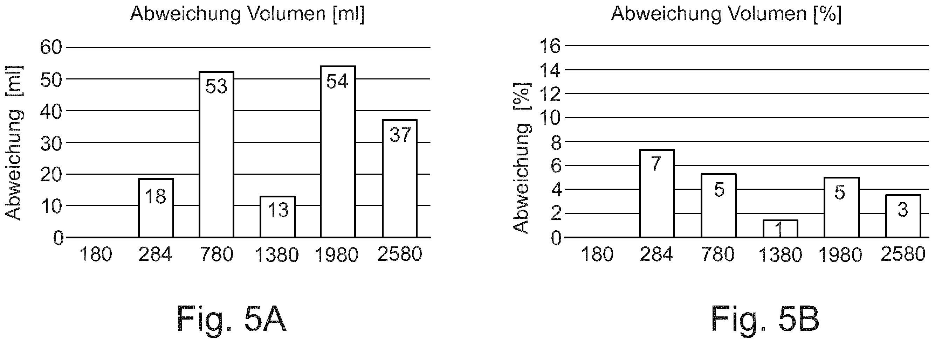

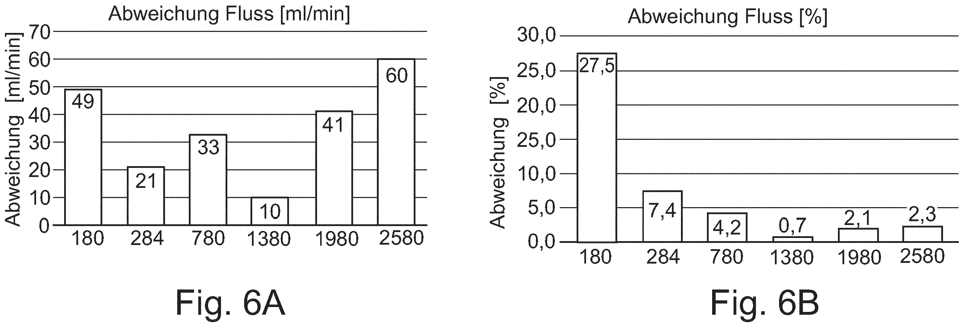

Figur 4 (A) und (B) stellt ein Messsignal des Ultraschall Durchflusssensors für einen Volumenstrom von 180 ml/min (Figur 4 (A)) und für einen Volumenstrom von 2560 ml/min (Figur 4(B)) dar. Nach dem Abstellen des Volumenstroms ist insbesondere bei höheren Flüssen ein kleines Nachschwingen der Flüssigkeitssäule im Messsignal zu erkennen. Die Messergebnisse für die verschiedenen konstanten Volumenströme sind in den Figuren 5 und 6 dargestellt. Die Messungen zeigen bei niedrigen Durchflüssen (180 ml/min und 284 ml/min) eine etwas höhere Abweichung der Durchflussmessung vom tatsächlichen Durchfluss. Bei höheren Durchflüssen reduziert sich der Messfehler allerdings konstant auf weniger als 5% (siehe Figur 6). Auch die Volumina lassen sich mit der erfindungsgemäßen Vorrichtung zuverlässig abbilden - hier verhalten sich die Fehler ähnlich wie bei der Durchflussmessung, was durch den direkten Zusammenhang von Fluss und Volumen auch zu erwarten war (siehe Figur 5). In den Figuren 5 und 6 ist jeweils in Figur (A) die absolute Abweichung der Messwerte einer Messung mit der erfindungsgemäßen Vorrichtung zu einer Wägemessung dargestellt und in Figur (B) die relative Abweichung der Messergebnisse.

Mit Hilfe der erfindungsgemäßen Vorrichtung ist eine verlässliche Bewertung eines Uriniervorgangs daher möglich. Der Messfehler bei niedrigen Volumenströmen (≤284 ml/min) ist immer noch ausreichend, um eine korrekte Diagnose im medizinischen Sinne zu stellen. Für Durchflüsse im höheren Volumenströmen (>284 ml/min) ist der Fehler mit < 5 % noch wesentlich geringer. In diesem Bereich ist auch die Wiedergabe einer aussagekräftigen Flusskennlinie möglich.

### Bezugszeichenliste

- 10: Einlauftrichter
- 11: ein Teil des Einlauftrichters
- 12: Auslauf des Einlauftrichters
- 20: Schlauch
- 30: Ultraschall Durchflusssensor
- 31: Datenkonverter
- 40: Ablassventil
- 50: Überlauf
- 51: nach oben gerichtete Auslassöffnung
- 60: Auffangbehälter
- 70: Stuhlkonstruktion
- 100: Vorrichtung

## Patentansprüche

1. Vorrichtung zum Messen eines Flusses einer Flüssigkeit in Abhängigkeit von der Zeit, insbesondere eines Urinflusses, aufweisend
• einen Einlauftrichter;
• einen Schlauch;
• einen Ultraschall Durchflusssensor;
• einen Überlauf;
**dadurch gekennzeichnet, dass**
ein erstes Ende des Schlauchs mit dem Einlauftrichter verbunden ist und ein zweites Ende des Schlauchs mit dem Überlauf;
der Ultraschall Durchflusssensor am Schlauch angeordnet ist;
der Schlauch U-förmig ausgebildet ist.

2. Vorrichtung gemäß Anspruch 1, **dadurch gekennzeichnet, dass** der Schlauch auf einer Länge von mindestens 10 x ID vor dem Ultraschall Durchflusssensor und auf einer Länge von mindestens 5 x ID hinter dem Ultraschall Durchflusssensor gerade verläuft.

3. Vorrichtung gemäß einem der vorangegangenen Ansprüche, **dadurch gekennzeichnet, dass** der Schlauch ein Polymerschlauch ist

4. Vorrichtung gemäß einem der vorangegangenen Ansprüche, **dadurch gekennzeichnet, dass** der Ultraschall Durchflusssensor ohne Koppelmittel am Schlauch angeklemmt ist.

5. Vorrichtung gemäß einem der vorangegangenen Ansprüche, **dadurch gekennzeichnet, dass** die Vorrichtung weiterhin ein Ablassventil aufweist, welches zwischen dem Ultraschall Durchflusssensor und dem Überlauf am Schlauch angeordnet ist.

6. Vorrichtung gemäß einem der vorangegangenen Ansprüche, **dadurch gekennzeichnet, dass** die Vorrichtung weiterhin einen Auffangbehälter aufweist, der nach dem Überlauf angeordnet ist.

7. Vorrichtung gemäß einem der vorangegangenen Ansprüche, **dadurch gekennzeichnet, dass** das zweite Ende des Schlauchs eine vertikale, nach oben gerichtete Auslassöffnung im Überlauf aufweist.

8. Vorrichtung gemäß einem der vorangegangenen Ansprüche, **dadurch gekennzeichnet, dass** der Einlauftrichter konzentrische auf den Ablauf zulaufenden Strömungsbrecher und/oder einen abgeknickten Ablauf aufweist.

9. Vorrichtung gemäß einem der vorangegangenen Ansprüche, **dadurch gekennzeichnet, dass** der Einlauftrichter eine Gitterstruktur im Auslauf des Trichters aufweist.

10. Vorrichtung gemäß einem der vorangegangenen Ansprüche, **dadurch gekennzeichnet, dass** die Vorrichtung weiterhin eine Batterie oder einen Akku aufweist.

11. Vorrichtung gemäß einem der vorangegangenen Ansprüche, **dadurch gekennzeichnet, dass** Teile der Vorrichtung oder die gesamte Vorrichtung weiterhin eine Umhüllung aufweisen.

12. Vorrichtung gemäß einem der vorangegangenen Ansprüche, **dadurch gekennzeichnet, dass** die Vorrichtung weiterhin eine Stuhlkonstruktion oder ein Urinal aufweist, wobei die Stuhlkonstruktion oder das Urinal mit dem Einlauftrichter verbunden ist, oder der Einlauftrichter durch die Stuhlkonstruktion oder das Urinal abgebildet wird, oder die genannte Vorrichtung in kompakter Bauform in einer Toilette eingehängt wird.
